# FASCICULE DE BREVET EUROPEEN

(11) **EP 2 465 939 B1**
(45) Date de publication et mention de la délivrance du brevet: **10.01.2018**
(21) Numéro de dépôt: 11188970.5
(22) Date de dépôt: 09.02.2006
(51) Int. Cl.: C12Q 1/04

(54) **Milieux pour la détection spécifique de microorganismes résistants**
Milieus zur spezifischen Erkennung von resistenten Mikroorganismen
Medium for the specific detection of resistant organisms

(30) Priorité: 10.02.2005 FR 0550394; 07.10.2005 FR 0553049
(43) Date de publication de la demande: 20.06.2012
(62) Demande divisionnaire de: 06709488.8
(73) Titulaire: bioMérieux, 69280 Marcy l'Etoile (FR)
(72) Inventeur: Orenga, Sylvain, 01160 Neuville Sur Ain (FR); Roger-Dalbert, CÉLINE, 01150 Chazey Sur Ain (FR); Perry, John, Newcastle upon Tyne, NE7 7BH (GB); Chanteperdrix, Vanessa, 48640 Varennes-Vauzelles (FR); Zambardi, Gilles, 38300 Chezeneuve (FR); Bal Brossard, Nathalie, 69280 Marcilly d'Azergues (FR)
(74) Mandataire: Bitaud, Valérie Marie-Odile

(56) Documents cités:
- FR-A1- 2 728 587
- FR-A1- 2 826 019
- US-A1- 2004 121 404
- PATTERSON T F ET AL: "Simple method for detecting fluconazole-resistant yeasts with chromogenic agar.", JOURNAL OF CLINICAL MICROBIOLOGY, vol. 34, no. 7, juillet 1996 (1996-07), pages 1794-1797, XP002678200, ISSN: 0095-1137
- MERLINO J ET AL: "ENZYMATIC CHROMOGENIC IDENTIFICATION AND DIFFERENTIATION OF ENTEROCOCCI", AUSTRALIAN JOURNAL OF MEDICAL SCIENCE, vol. 19, no. 3, 1 août 1998 (1998-08-01), pages 76-81, XP008016751, AUSTRALIAN INSTITUTE OF MEDICAL SCIENTISTS, TOOWONG, AU ISSN: 1038-1643

## Description

Le domaine de l'invention est celui de l'analyse microbiologique par voie biochimique, et en particulier de la détection et de l'identification de microorganismes, tels que notamment de bactéries ou de levures.

La résistance des bactéries aux antibiotiques est un problème majeur de santé publique. La résistance de microorganismes infectieux envers un traitement s'est développée en même temps que les molécules anti-infectieuses et représente aujourd'hui un obstacle majeur en thérapeutique. Cette résistance est source de problèmes multiples, incluant les difficultés de détection au laboratoire, les options de traitement limitées et un impact délétère sur l'issue clinique.

En particulier, l'augmentation rapide et irrépressible de la résistance des bactéries pathogènes, au cours des 20 dernières années, représente un des problèmes actuels majeurs de la médecine. Les infections causées par ces organismes sont à l'origine de l'allongement de la durée d'hospitalisation et sont associées à des taux élevés de morbidité et de mortalité, suite à des échecs thérapeutiques.

Plusieurs mécanismes de résistance peuvent être impliqués simultanément chez une souche bactérienne. Ils se classent en général en 3 catégories : défaut de pénétration de l'antibiotique dans la bactérie, inactivation ou excrétion de l'antibiotique par des systèmes enzymatiques bactériens et défaut d'affinité entre la cible bactérienne et l'antibiotique.

L'inactivation enzymatique est le mécanisme le plus fréquent de résistance acquise en termes de nombre d'espèces et d'antibiotiques concernés. Ainsi, les céphalosporinases chromosomiques de classe C constituent aujourd'hui un des mécanismes de résistance prédominants des bactéries gram-négatif, les bactéries exprimant de telles enzymes étant résistantes aux céphalosporines. De même, les β-lactamases sont des enzymes exprimées par certaines bactéries, capables d'hydrolyser la liaison C-N du noyau β-lactame, structure de base des antibiotiques de la famille des β-lactamines, pour donner un produit microbiologiquement inactif. Plusieurs inhibiteurs de β-lactamases (IBL), tels que l'acide clavulanique (AC), le tazobactam et le sulbactam, ont été développés pour augmenter l'activité antimicrobienne et élargir le spectre des β-lactamines qui leur sont associées. Agissant comme un substrat suicide des β-lactamases, ils empêchent la dégradation enzymatique des antibiotiques et leur permettent de devenir efficaces contre des bactéries initialement résistantes. Cependant, de par l'exposition persistante des souches à la pression antibiotique, les bactéries expriment leur capacité d'adaptation par la production continue et dynamique de β-lactamases, évoluant en même temps que le développement de nouvelles molécules.

Les bactéries gram-négatif productrices de céphalosporinases chromosomiques de classe C de haut niveau (on parle de bactéries Case HN), ainsi que les bactéries gram négatif productrices de β-lactamases à spectre étendu (on parle alors de bactéries BLSE) sont devenues de ce fait une menace grandissante, en particulier parce que le nombre d'espèces bactériennes concernées augmente. Les bactéries Case HN et BLSE sont résistantes à des traitements à base de pénicillines et céphalosporines de 1ère et 2ème générations, mais également de céphalosporines de 3ème génération (C3G) (cefotaxime CTX, ceftazidime CAZ, cefpodoxime CPD, ceftriaxone CRO) et des monobactams (aztreonam ATM). Par contre, les 7α-méthoxycéphalosporines (céphamycines: cefoxitine, cefotetan) et les carbapénèmes (imipénème, méropénème, ertapénème) conservent généralement leur activité. Les BLSE sont inhibées par les inhibiteurs de β-lactamases (IBL), ce qui permet de les différencier des autres céphalosporinases.

Ces bactéries expriment ainsi le plus souvent simultanément des résistances à plusieurs traitements, ce qui pose des difficultés pour installer un traitement pertinent et éviter les échecs thérapeutiques. Une bactérie *Escherichia coli* peut ainsi être Case HN et BLSE. En outre, comme les entérobactéries BLSE positives ont tendance à disséminer la résistance par transmission clonale de souches ou transfert plasmidique conjugatif, elles représentent un problème pour le contrôle des infections. Dans la plupart des études, *Escherichia coli* et *Klebsiella pneumoniae* demeurent les espèces productrices de BLSE les plus communes. Mais les BLSE ont, depuis quelques années, grandement élargi leur panel d'espèces hôtes. En effet, de nombreuses espèces d'entérobactéries et de bacilles gram-négatif non fermentants (comme *Pseudomonas aeruginosa*) ont également été rapportés comme producteurs de BLSE.

Outre ces bactéries BLSE, on peut également citer les bactéries *Staphylococcus aureus* qui sont également des bactéries pathogènes développant de nombreux mécanismes de résistances, telles qu'une résistance à la méticilline, la pénicilline, la tétracycline, l'érythromycine, la vancomycine. *Enterococcus faecium* est une autre bactérie multirésistante trouvée en milieu hospitalier, qui peut être résistante à la pénicilline, la vancomycine et au linezolide. *Mycobacterium tuberculoses* est couramment résistant à l'isoniazide et au rifampicine. D'autres pathogènes offrent certaines résistances comme *Salmonella, Campylobacter* et *Streptococcus.*

Il devient donc indispensable, d'un point de vue de santé publique, de pouvoir identifier le plus rapidement possible de tels microorganismes, et de tels mécanismes de résistances.

En général, la recherche des micro-organismes résistants à un traitement se fait selon les étapes suivantes
1. prélèvement d'un échantillon biologique susceptible de contenir lesdits micro-organismes
2. ensemencement et incubation d'un milieu de culture (18 à 48H) pour induire une croissance exponentielle des micro-organismes
3. Repérage sur les milieux de culture des colonies de micro-organismes potentiellement significatifs
4. Caractérisation de l'espèce de micro-organisme
5. Identification des mécanismes de résistance des micro-organismes analysés, leur signification biologique et éventuellement la thérapie adaptée.

Cette succession d'étapes implique un temps important entre le prélèvement de l'échantillon susceptible de contenir des micro-organismes, et la prescription d'un traitement adapté pour le patient. De plus, l'utilisateur doit généralement réaliser manuellement des étapes de transfert de micro-organismes d'un premier milieu vers un deuxième milieu, ce qui peut induire des problèmes notamment de contamination mais également des risques pour la santé du manipulateur.

A titre d'exemple, pour détecter la présence de béta-lactamases à spectre étendu (BLSE) dans des souches d'*Escherichia coli* et de *Klebsiella pneumoniae,* on peut utiliser une technique de diffusion telle que décrite dans la publication de Jacoby & Han (J Clin Microbiol. 34(4): 908-11, 1996) qui ne donne toutefois aucune information sur l'identification des souches testées : on peut déterminer si la bactéries est une bactérie productrice de BLSE ou non, mais on ne peut distinguer si une telle bactérie est une *Escherichia coli* ou une *Klebsiella pneumoniae.* Des substrats métaboliques sont également utilisés pour détecter la présence de BLSE ou Case HN. A ce titre, les laboratoires AES proposent un milieu dans une bi boîte associant un milieu Drigalski avec du céfotaxime et un milieu MacConkey avec de la Ceftazidime. Les milieux Drigalski et MacConkey permettent de révéler l'acidification du Lactose, métabolisme présent chez un très grand nombre d'espèces d'entérobactéries. Toutefois, un tel milieu permet uniquement de distinguer des bactéries résistantes de bactéries non résistantes, mais ne permet pas de distinguer les bactéries exprimant une BLSE de celles exprimant une Case HN. Ce milieu ne permet pas non plus l'identification d'espèces particulières de bactéries, et ne permet pas, par exemple de discriminer les bactéries *E. coli,* de bactéries *K. pneumoniae.* Dans le cas de la détection de mécanismes de résistance autre que BLSE, on peut citer la demande de brevet EP0954560 qui concerne la recherche des entérocoques résistants à la Vancomycine, en combinant la Vancomycine avec un milieu chromogène révélant deux activités enzymatiques (β-glucosidase et pyrrolidonyl-arylamidase). Toutefois, ce milieu chromogène permet de déterminer uniquement si les souches résistantes à la vancomycine appartiennent ou n'appartiennent pas au genre *Enterococcus,* mais ne permet pas d'identifier l'espèce ou les mécanismes de résistance impliqués, notamment s'il s'agit d'une résistance acquise ou sauvage.

Ainsi, la caractérisation d'une espèce de microorganisme, puis l'identification de sa résistance à un traitement, est longue et fastidieuse. Si le laboratoire rend au clinicien un dépistage positif alors que l'isolat est en fait dépourvu de microorganismes résistants, cela peut entraîner un traitement inutile et inadapté. Inversement, ne pas communiquer un dépistage positif qui sera ensuite confirmé retarde la mise en place de l'isolement du patient (et éventuellement d'une thérapeutique adaptée) d'une journée. Cela montre le besoin d'un test de confirmation rapide et fiable.

La présente invention se propose donc d'améliorer l'état de la technique en présentant un nouvel outil de diagnostic permettant un gain de temps, de fiabilité et de pertinence dans la thérapie mise en oeuvre. Notre invention permet en une seule étape d'identifier les espèces de micro-organismes présents dans un échantillon, déterminer leur mécanisme de résistance afin de proposer un traitement adapté à chaque patient. Cette invention est particulièrement adaptée pour discriminer différentes espèces de microorganismes, présentant différents mécanismes de résistance à différent traitement, mais toutes susceptible d'être présentes dans un même échantillon.

Avant d'aller plus avant dans l'exposé de l'invention, les définitions suivantes sont données afin de faciliter la compréhension de l'invention :
Par milieu de culture, on entend un milieu comprenant tous les éléments nécessaires à la survie et/ou à la croissance de microorganismes. Le milieu de culture selon l'invention peut contenir d'éventuels autres additifs comme par exemple: des peptones, un ou plusieurs facteurs de croissance, des hydrates de carbone, un ou plusieurs agents sélectifs, des tampons, un ou plusieurs gélifiants... Ce milieu de culture peut se présenter sous forme de liquide, de gel prêt à l'emploi, c'est à dire prêt à l'ensemencement en tube, flacon, ou sur boite de Petri.

Au sens de la présente invention, le terme microorganisme recouvre les bactéries, gram positif ou gram négatif, les levures et plus généralement, les organismes généralement unicellulaires, invisibles à l'oeil nu, qui peuvent être multipliés et manipulés en laboratoire.

A titre de bactéries Gram négatif, on peut citer les bactéries des genres suivants: *Pseudomonas, Escherichia, Salmonella, Shigella, Enterobacter, Klebsiella, Serratia, Proteus, Campylobacter, Haemophilus, Morganella, Vibrio, Yersinia, Acinetobacter, Branhamella, Neisseria, Burkholderia, Nitrobacter, Hafnia, Edwardsiella, Aeromonas, Moraxella, Pasteurella, Providencia, et Legionella.*

A titre de bactéries Gram positif, on peut citer les bactéries des genres suivants: *Enterococcus, Streptococcus, Staphylococcus, Bacillus, Listeria, Clostridium, Gardnerella, Kocuria, Lactococcus, Leuconostoc, Micrococcus, Mycobacteria et Corynebacteria.*

A titre de levures, on peut citer les levures des genres suivants: *Candida, Cryptococcus, Saccharomyces et Trichosporon.*

Par échantillon biologique, on entend un échantillon clinique, issu d'un prélèvement de liquide biologique, ou un échantillon alimentaire, issu de tout type d'aliment. Cet échantillon peut être ainsi liquide ou solide et on peut citer d'une manière non limitative, un échantillon clinique de sang, de plasma, d'urines, de fécès, de prélèvements de nez, de gorges, de peaux, de plaies, de liquide céphalo-rachidien, un échantillon alimentaire d'eau, de boissons tels que le lait, un jus de fruits; de yaourt, de viande, d'oeufs, de légumes, de mayonnaise, de fromage ; de poisson..., un échantillon alimentaire issu d'une alimentation destinée aux animaux, tel que notamment un échantillon issu de farines animales.

Par mécanisme de résistance, on entend tout type de dispositif qui permet à un micro-organisme de rendre un traitement partiellement ou totalement inopérant sur ledit microorganisme, garantissant sa survie. Les mécanismes de résistance se classent en général en 3 catégories : défaut de pénétration de l'antibiotique dans la bactérie, inactivation ou excrétion de l'antibiotique par des systèmes enzymatiques bactériens et défaut d'affinité entre la cible bactérienne et l'antibiotique.

A titre indicatif, on peut citer notamment les mécanismes de résistance liés à l'expression d'une enzyme appartenant au groupe des β-lactamases à spectre étendu ; d'une enzyme appartenant au groupe des céphalosporinases chromosomiques de classe C haut niveau ; les mécanismes de résistance aux glycopeptides, préférentiellement développés par des bactéries appartenant au genre *Enterococcus.*

On citera également les mécanismes de résistance à la méticilline, la pénicilline, la tétracycline, l'érythromycine, la vancomycine lorsque le microorganismes est une bactérie

### Staphylococcus aureus

On citera également les mécanismes de résistance à la pénicilline, la vancomycine et au linezolide lorsque le microorganismes est une bactérie *Enterococcus faecium*.

On citera également les mécanismes de résistance à l'amphotéricine B ou aux antifongiques de la famille des azolés lorsque le microorganisme est une levure.

On citera enfin les mécanismes de résistance à l'isoniazide et au rifampicine lorsque le microorganismes est une bactérie *Mycobacterium tuberculosis.*

Par traitement, on entend un traitement susceptible d'empêcher ou de réduire la croissance de microorganismes issus d'un patient. Ce traitement peut comprendre notamment des composés antimicrobiens, tels que des antibiotiques, tels que pénicillines, céphalosporines classiques, céphalosporines à spectre étendu, monobactams, glycopeptides, aminosides ou tels que des antifongiques ou des composés inhibiteurs de la résistance. A noter que ce traitement peut comprendre en outre l'isolement du patient, ce qui empêche la propagation du microorganisme au sein d'autres patients.

Par substrat permettant la détection d'une activité enzymatique ou métabolique, on entend toute molécule susceptible d'engendrer directement ou indirectement un signal détectable dû à une activité enzymatique ou métabolique du microorganisme.

Lorsque cette activité est une activité enzymatique, on parle alors de substrat enzymatique. Par substrat enzymatique, on entend tout substrat pouvant être hydrolysé par une enzyme en un produit permettant la détection, directe ou indirecte d'un microorganisme. Ce substrat comprend notamment une première partie spécifique de l'activité enzymatique à révéler et une seconde partie faisant office de marqueur, ci-après appelée partie marqueur. Cette partie marqueur peut être chromogène, fluorogène, luminescente... Comme substrat chromogène, bien adapté aux supports solides (filtre, gélose, gel d'électrophorèse), on peut citer notamment les substrats à base d'indoxyl et ses dérivés, et les substrats à base d'hydroxyquinoline ou d'esculétine et leurs dérivés, qui permettent la détection d'activités osidase et estérase. On peut citer également les substrats à base de nitrophénol et nitroaniline et dérivés, permettant de détecter les activités osidases et estérases dans le cas de substrats à base de nitrophénol, et des activités peptidases dans le cas de substrats à base de la nitroaniline. On peut citer enfin les substrats à base de naphtol et naphtylamine et leurs dérivés, qui permettent de détecter les activités osidases et estérases par l'intermédiaire du naphtol, et les activités peptidases par l'intermédiaire de la naphtylamine. Ce substrat peut permettre notamment, mais d'une façon non limitative, la détection d'une activité enzymatique telle que l'activité d'une osidase, peptidase, estérase... Le substrat enzymatique peut également être un substrat naturel dont le produit d'hydrolyse est détecté directement ou indirectement. Comme substrat naturel, on peut notamment citer le Tryptophane pour détecter une activité tryptophanase ou desaminase, un acide aminé cyclique (Tryptophane, Phénylalanine, Histidine, Tyrosine) pour détecter une activité désaminase, le Phosphatidyl Inositol pour détecter une activité phospholipase, ... Lorsque cette activité est une activité métabolique, le substrat est alors un substrat métabolique, telle qu'une source de carbone ou d'azote, couplée à un indicateur produisant une coloration en présence de l'un des produits du métabolisme.

Selon un mode préféré de réalisation de l'invention, ladite première et/ou deuxième activité enzymatique ou métabolique est une activité enzymatique, préférentiellement choisie parmi les activités enzymatiques : beta-glucosidase, desaminase, beta-glucuronidase, beta-galactosidase, alpha-glucosidase, alpha-galactosidase, hexosaminidase, N-acétyl-hexosaminidase, phosphatase, estérase, aminopeptidase.

Par exemple, pour détecter *E. coli,* on utilise préférentiellement l'activité beta-glucuronidase ou β-galactosidase, ou tryptophanase ou desaminase ; pour détecter les *Proteus* on utilise préférentiellement l'activité désaminase, pour détecter les entérocoques, on utilise préférentiellement l'activité beta-glucosidase. Pour les *Candida albicans,* on privilégie l'hexosaminidase, pour les *Listeria monocytogenes* la phospholipase, pour les salmonelles l'estérase, pour les *Pseudomonas aeruginosa* l'estérase ou la β-alanine aminopeptidase, pour les *Staphylococcus aureus* la phosphatase ou l'alpha-glucosidase.

Par marqueur permettant la différenciation de deux groupes de microorganismes, on entend un composé qui n'a pas les mêmes propriétés sur un premier et sur un deuxième groupe. Ce composé peut être ainsi :
- un substrat spécifique
- un inhibiteur de mécanisme de résistance, qui permet alors d'inhiber la croissance des organismes développant une résistance particulière, sans discrimination de l'espèce de microorganismes

Dans le cas de l'utilisation d'un substrat spécifique, on utilise préférentiellement l'activité beta-glucuronidase, ou beta-galactosidase ou tryptophanaseou desaminase pour détecter *E. coli,* pour détecter les *Proteus* on utilise préférentiellement l'activité désaminase, pour détecter les entérocoques, on utilise préférentiellement l'activité beta-glucosidase. Pour les *Candida albicans,* on privilégie l'hexosaminidase, pour les *Listera monocytogenes* la phospholipase, pour les salmonelles l'estérase, pour les *Pseudomonas aeruginosa* l'estérase ou la β-alanine aminopeptidase, pour les *Staphylococcus aureus* la phosphatase ou l'alpha-glucosidase.

Dans le cas de l'utilisation d'un inhibiteur de mécanisme de résistance, on utilise préférentiellement
- l'acide clavulanique, le tazobactam, ou le sulbactam lorsque le premier groupe et/ou le deuxième groupe comprend un mécanisme de résistance induit par l'expression de β-lactamases. La concentration en acide clavulanique dans le milieu est alors préférentiellement comprise entre 0,05 et 32 mg/l, préférentiellement entre 0,1 et 8 mg/l et encore plus préférentiellement entre 0,25 et 6mg/l.
- la cloxacilline ou la dicloxacilline lorsque le premier groupe et/ou le deuxième groupe comprend un mécanisme de résistance induit par l'expression de céphalosporinases
Par taxon, on entend un groupe de microorganismes ayant une unité taxonomique. Un taxon peut être une famille, un genre, un ensemble de genres, une espèce, un ensemble d'espèces, une sous-espèce. A titre indicatif on peut citer les entérobactéries, les *Klebsiella,* les *Escherichia,* les *Enterobacter,* les *Citrobacter,* les *Serratia,* les KESC (*Klebsiella, Enterobacter, Serratia, Citrobacter*), les *Proteeae,* les *Proteus,* les *Morganella,* les *Pseudomonas,* les *Staphylococcus,* les *Streptococcus,* les *Enterococcus,* les *Candida, Escherichia coli, Escherichia coli* O157:H7, *Klebsiella pneumoniae, Citrobacter freundii*, *Enterococcus faecalis, Enterococcus faecium, Staphylococcus aureus,* les staphylocoques à coagulase négative, *Candida albicans, Candida glabrata, Candida krusei, Candida lusitaniae.*

Par antimicrobien, on entend tout composé susceptible d'empêcher ou de ralentir la croissance d'un microorganisme. Ce composé peut être un antibiotique ou un antifongique.

Par antibiotique, on entend tout composé susceptible d'empêcher ou de ralentir la croissance d'une bactérie. A titre indicatif, on peut citer notamment les antibiotiques cefotaxime, ceftazidime, ceftriaxone, cefpodoxime, aztréonam, vancomycine, tobramycine, ciprofloxacine.

Par antifongiques, on entend tout composé susceptible d'empêcher ou de ralentir la croissance d'une levure ou d'une moisissure. A titre indicatif, on peut citer notamment l'amphotéricine B, le fluconazole, l'itraconazole, le voriconazole, la cycloheximide.

Selon un mode préféré de réalisation de l'invention, lorsque l'antibiotique est
- la cefotaxime, la concentration en cefotaxime dans le milieu est préférentiellement comprise entre 0,25 et 8 mg/l, préférentiellement entre 1 et 2 mg/l.
- la ceftazidime, la concentration en ceftazidime dans le milieu est préférentiellement comprise entre 0,25 et 8 mg/l, préférentiellement entre 2 et 2,5 mg/l.
- la ceftriaxone, la concentration en ceftriaxone dans le milieu est préférentiellement comprise entre 0,25 et 8 mg/l, préférentiellement entre 1 et 2,5 mg/l.
- la cefpodoxime, la concentration en cefpodoxime dans le milieu est préférentiellement comprise entre 0,1 et 32 mg/l, préférentiellement entre 0,75 et 10 mg/l et encore plus préférentiellement entre 1 et 6 mg/l.
- l'aztréonam, la concentration en aztréonam dans le milieu est préférentiellement comprise entre 0,1 et 8 mg/l, préférentiellement entre 0,75 et 1,5 mg/l.

Selon un mode particulier de réalisation de l'invention, le milieu comprend une combinaison d'au moins deux antibiotiques. Préférentiellement, la combinaison d'au moins deux antibiotiques comprend cefotaxime et ceftazidime.

Quelque soit le mode de réalisation de l'invention, le milieu peut comprendre en outre un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant...

Tous les milieux peuvent comprendre, en outre, afin d'augmenter leur sensibilité
- au moins un antimicrobien actif contre les bactéries gram positif, tel que notamment le linezolide ou la vancomycine.
- au moins un antimicrobien actif contre les levures, tel que notamment le voriconazole ou l'amphotéricine B.

L'invention concerne l'utilisation d'un milieu de culture pour distinguer au moins 3 groupes de microorganismes dans un échantillon biologique comprenant :
- un premier groupe de microorganismes, appartenant à un premier taxon de microorganismes et comprenant au moins un mécanisme de résistance à un traitement
- un deuxième groupe de microorganismes, appartenant à un deuxième taxon de microorganismes, différent dudit premier taxon, mais comprenant au moins un mécanisme de résistance à un traitement, identique à celui du premier groupe
- un troisième groupe de microorganismes, non résistants audit traitement ledit milieu de culture comprenant
   a. au moins un premier substrat permettant la détection d'au moins une première activité enzymatique ou métabolique dudit premier groupe de microorganismes
   b. au moins un marqueur permettant la différenciation du premier groupe de microorganismes et du deuxième groupe de microorganismes, ledit marqueur étant un substrat permettant la détection d'au moins une activité enzymatique ou métabolique dudit deuxième groupe de microorganismes
   c. au moins un antimicrobien, actif sur ledit troisième groupe de microorganismes

Ce mode de réalisation de l'invention permet de distinguer dans un même échantillon, un premier et un deuxième groupe comprenant différentes espèces ou différents taxons de microorganismes, mais chacun des deux groupes étant résistants à un même traitement.

Ce mode de réalisation de l'invention permet ainsi, par exemple de distinguer, dans un même échantillon, un premier groupe comprenant des bactéries *E coli* BLSE, et un deuxième groupe comprenant des bactéries KESC BLSE.

L'invention concerne un milieu de culture comprenant
- un premier substrat permettant la détection d'une activité enzymatique beta glucuronidase ou beta galactosidase, préférentiellement 6-Chloro-3-indolyl-β-D-glucuronide à une concentration comprise entre 25 et 750mg/l, préférentiellement entre 40 et 300mg/l ou 5-Bromo-6-chloro-3-indolyl-β-D-galactoside à une concentration comprise entre 25 et 500mg/l, préférentiellement entre 40 et 150mg/l
- un deuxième substrat permettant la détection d'une activité beta glucosidase, préférentiellement le 5-Bromo-4-chloro-3-indolyl-β-D-glucoside à une concentration comprise entre 25 et 500 mg/l, préférentiellement entre 40 et 250 mg/l ou tryptophanase ou desaminase, préférentiellement le Tryptophane à uneconcentration comprise entre 50 et 5000 mg/l, préférentiellement entre 250 et 2000 mg/l
- un antimicrobien qui est un antibiotique, préférentiellement la cefpodoxime à une concentration comprise entre 0,5 et 32 mg/l, préférentiellement entre 0,75 et 10 mg/l et encore plus préférentiellement entre 1 et 6 mg/l et de la cloxacilline à une concentration comprise entre 10 et 2000 mg/l, préférentiellement entre 50 et 500 mg/l

Lorsque l'antibiotique est la cefpodoxime, ce milieu est préférentiellement utilisé pour distinguer:
- un premier groupe de bactéries *E. coli* BLSE
- un deuxième groupe de bactéries KESC BLSE
- un troisième groupe de bactéries, non résistantes aux beta lactamines

L'invention concerne également un milieu de culture comprenant :
- un premier substrat permettant la détection d'une activité enzymatique beta glucuronidase, préférentiellement 6-Chloro-3-indolyl-β-D-glucuronide à une concentration comprise entre 25 et 750 mg/l, préférentiellement entre 40 et 300 mg/l ou beta galactosidase, préférentiellement 5-Bromo-6-chloro-3-indolyl-β-D-galactoside à une concentration comprise entre 25 et 500 mg/l, préférentiellement entre 40 et 150 mg/l
- un deuxième substrat permettant la détection d'une activité beta glucosidase, préférentiellement le 5-Bromo-4-chloro-3-indolyl-β-D-glucoside à une concentration comprise entre 25 et 500mg/l, préférentiellement entre 40 et 250mg/l ou tryptophanase ou desaminase, préférentiellement le Tryptophane à une concentration comprise entre 50 et 5000mg/l, préférentiellement entre 250 et 2000mg/l
- deux antimicrobiens, qui sont préférentiellement deux antibiotiques, préférentiellement de la cefpodoxime à une concentration comprise entre 0,1 et 32 mg/l, préférentiellement entre 0,25 et 10 mg/l et encore plus préférentiellement entre 0,5 et 4 mg/l et·de l'aztreonam à une concentration comprise entre 0,1 et 8 mg/l, préférentiellement entre 0,5 et 1,5 mg/l

Ce milieu est préférentiellement utilisé pour distinguer:
- un premier groupe de bactéries *E. coli* BLSE ou Case NH
- un deuxième groupe de bactéries KESC BLSE ou Case NH
- un troisième groupe de bactéries, non résistantes aux beta lactamines et/ou aux céphalosporines

L'invention concerne également un milieu de culture comprenant :
- un antimicrobien, préférentiellement un antibiotique tel que la cloxacilline
- un inhibiteur de mécanisme de résistance, tel que préférentiellement une céphalosporine de 3ème génération choisie parmi cefotaxime, ceftazidime, cefpodoxime, ceftriaxone

Ce milieu est également préférentiellement utilisé pour détecter des bactéries BLSE

Le mode de réalisation de l'invention décrit *supra* n'est pas limité à la distinction de 3 groupes de microorganismes, mais peut permettre la distinction de 4, 5 ou même plus, groupes de microorganismes. Il est alors nécessaire de rajouter dans le milieu des marqueurs d'identification entre les différents groupes.

A ce titre, l'invention concerne également un milieu de culture comprenant :
- un premier substrat permettant la détection d'une activité enzymatique beta glucuronidase, préférentiellement 6-Chloro-3-indolyl-β-D-glucuronide à une concentration comprise entre 25 et 750mg/l, préférentiellement entre 40 et 300mg/l ou béta galactosidase, préférentiellement 5-Bromo-6-chloro-3-indolyl-β-D-galactoside à une concentration comprise entre 25 et 500mg/l, préférentiellement entre 40 et 150m/l
- un deuxième substrat permettant la détection d'une beta glucosidase, préférentiellement le 5-Bromo-4-chloro-3-indolyl-β-D-glucoside à une concentration comprise entre 25 et 500mg/l, préférentiellement entre 40 et 250mg/l
- une combinaison d'antimicrobien, préférentiellement une combinaison d'antibiotiques tel que
   ∘ la cefpodoxime à une concentration comprise entre 0,5 et 32 mg/l, préférentiellement entre 0,75 et 10 mg/l et encore plus préférentiellement entre 1 et 6 mg/l,
   ∘ la cloxacilline à une concentration comprise entre 10 et 2000mg/l, préférentiellement entre 50 et 500mg/l,
   ∘ la vancomycine à une concentration comprise entre 0,5 et 128mg/l, préférentiellement entre 2 et 32mg/l et·
   ∘ l'ampho B à une concentration comprise entre 0,5 et 64mg/l, préférentiellement entre 1 et 16mg/l, encore plus préférentiellement entre 1 et 8mg/l
- un troisième substrat permettant la détection d'une activité désaminase telle que l'Histidine, la Phénylalanine, le Tryptophane ou la Tyrosine à une concentration comprise entre 50 et 5000 mg/l, préférentiellement entre 250 et 2000 mg/l

Ce milieu peut comprendre en outre un cinquième antibiotique qui est le cefsulodine, à une concentration comprise entre 0,5 et 64mg/l, préférentiellement entre 1 et 16mg/l.

Ce milieu est préférentiellement utilisé pour distinguer:
- un premier groupe de bactéries *E. coli* BLSE
- un deuxième groupe de bactéries KESC BLSE
- un troisième groupe de bactéries, non résistantes aux beta lactamines et/ou aux cephalosporines
- un quatrième groupe de bactéries *Proteeae* BLSE

Par l'utilisation d'une combinaison d'antimicrobiens adaptée, il est possible de distinguer non seulement trois groupes de microorganismes mais aussi 4, 5 ou même plus, groupes de microorganismes.

Les exemples ci dessous sont donnés à titre explicatif et n'ont aucun caractère limitatif. Ils permettront de mieux comprendre l'invention.

### EXEMPLE 1

L'exemple ci dessous est basé sur la détection phénotypique des BLSE utilisant la réduction de susceptibilité de ces souches aux antibiotiques et leur sensibilité aux associations avec les inhibiteurs de β-lactamases. Pour cela, une bi-boîte en base CPS ID 3 (milieu chromogène pour la détection des micro-organismes dans les urines et vendus par bioMérieux sous la référence 43541) avec une demi-gélose contenant un antibiotique et une demi-gélose contenant une association antibiotique - inhibiteur de β-lactamases a été utilisée.

### 1. Choix des souches

Dans le cadre des manipulations effectuées, pour l'évaluation de l'activité des antibiotiques actifs sur les bacilles gram-négatif, différentes espèces d'entérobactéries (*Escherichia coli, Enterobacter aerogenes, Klebsiella pneumoniae, Serratia marcescens, Proteus mirabilis*) et de bacilles gram-négatif non fermentants (*Pseudomonas aeruginosa*) susceptibles de produire des BLSE ont été employées. Dans les essais sont comparées des souches BLSE positives, des souches productrices de céphalosporinase haut niveau (Case HN) et des souches sauvages.

Pour les manipulations concernant les antibiotiques actifs sur les bactéries gram-positif, des souches de cocci gram-positif (*Staphylococcus aureus, Staphylococcus saprophyticus, Staphylococcus epidermidis, Enterococcus faecalis, Enterococcus faecium, Streptococcus agalactiae*) et de bacilles gram-positif (*Lactobacillus spp*) ont été testées.

Pour les essais destinés à évaluer l'activité des antifongiques, différentes souches de levures (*Candida albicans, Candida glabrata, Candida tropicalis, Candida krusei, Candida dubliniensis, Saccharomyces cerevisiae, Geotrichum capitatum*) ont été utilisées.

### 2. Préparation des milieux

Le milieu utilisé était un milieu CPS ID3 (référence 43541) comprenant en outre au moins un antibiotique et au moins un inhibiteur de mécanisme de résistance.
La composition des milieu testés était la suivante :

| | Substrat chromogénique | Antibiotique | Inhibiteur de résistance |
|---|---|---|---|
| Milieu A | 6-chloro-3-indolyl-β-D-glucuronide | Cefotaxime 1mg/l | |
| | 5-Bromo-4-chloro-3-indolyl-β-D-glucoside | | |
| | Tryptophane | | |
| | FeCl₃ | | |
| | Total : 1,73g/l | | |
| Milieu B | 6-chloro-3-indolyl-β-D-glucuronide | Ceftazidime 1,5mg/l | Ac clavulanique 0,25mg/l |
| | 5-Bromo-4-chloro-3-indolyl-β-D-glucoside | | |
| | Tryptophane | | |
| | FeCl₃ | | |
| | Total : 1,73g/l | | |

L'eau osmosée est ajoutée et le tout est homogénéisé et mis à fondre au bain-marie à 100°C. Le milieu de base est réparti en flacons, en nombre correspondant au nombre total de milieux à tester lors de la manipulation. Les flacons sont ensuite autoclavés 15 mn à 121°C. Les milieux sont ramenés et maintenus en surfusion à 55 ± 3°C au bain-marie, afin d'ajouter stérilement les additifs thermolabiles (stérilisés préalablement par filtration sur 0,22 µm). Les milieux sont ensuite coulés en bi-boîtes de 90 mm de diamètre (soit environ 9,5 mL/demi-boîte), et laissés sur une surface plane pour qu'ils puissent reprendre en masse. Puis la surface des géloses est séchée sous hotte à flux laminaire pendant 30 mn.

### 3. Ensemencement des milieux

A partir de précultures de 24 heures à 36°C ± 2°C en atmosphère aérobie sur milieu TSA, un inoculum de 0,5 McF est réalisé en eau physiologique, puis 1 µL de cette suspension est transféré dans 5 mL d'eau physiologique. Afin d'obtenir des colonies isolées suffisamment nombreuses, une gamme d'inocula a permis de déterminer que la quantité optimale de bactéries à ensemencer était de 10³ à 10⁴ CFU/mL. L'ensemencement se fait directement sur les 2 demi-géloses à l'aide d'un écouvillon stérile. Puis les cultures sont incubées à 37°C en atmosphère aérobie.

**4. Lecture des milieux :** Les lectures sont effectuées à 18 heures (± 30 min), 24h (± 1h) et 48h (± 4h). Ont été observés la densité et la taille des colonies, l'aspect, la couleur et les intensités de coloration de la masse et des colonies isolées, selon les échelles de lecture 1 à 3 suivantes : 0 : pas de croissance; 0,1: trace de pousse; 0,25: colonies de diamètre < 0,5 mm; 0,5 : colonies de 0,5 mm diamètre; 0,75 : 0,5 mm < diamètre < 1 mm; 1: colonies de 1 mm diamètre; 1,25: 1 mm < diamètre < 1,5 mm; 1,5: colonies de 1,5 mm diamètre; 2: colonies de 2 mm diamètre; 3: colonies de diamètre > 2 mm.

### 5. Résultats :

### a) Screening des antibiotiques

Les 5 antibiotiques recommandés par le NCCLS (National Committee for Clinical Laboratory Standards) ont été testés. Pour chaque produit testé, une gamme a été effectuée afin de déterminer les concentrations permettant d'inhiber les souches sauvages des entérobactéries testées, sans affecter la croissance des souches BLSE positives ni les souches Case HN positives.

Les concentrations retenues sont répertoriées dans le tableau III ci-après.

**Tableau III. Valeurs limites des concentrations d'antibiotiques permettant une inhibition des souches sauvages des entérobactéries testées, sans affecter la croissance des souches BLSE-positives dans les conditions testées**

| | valeur basse | valeur haute |
|---|---|---|
| Cefotaxime | 1 mg/L | 2 mg/L |
| Ceftazidime | 2 mg/L | 2,5 mg/L |
| Ceftriaxone | 1 mg/L | 2,5 mg/L |
| Cefpodoxime | 2 mg/L | 10 mg/L |
| Aztreonam | 1 mg/L | 1,5 mg/L |

La valeur basse correspondant à la concentration minimale de l'antibiotique nécessaire à l'inhibition des souches sauvages des entérobactéries testées. La valeur haute correspond à la concentration maximale de l'antibiotique pouvant être utilisée sans affecter la croissance des souches BLSE positives testées.

A ces concentrations, la séparation des souches sauvages et résistantes est satisfaisante, et l'expression des activités enzymatiques sur le milieu CPS ID 3 est conforme.

En outre, il est à noter que la ceftazidime est le seul antibiotique testé et utilisé dans la détection des BLSE qui ait montré une activité sur les souches sauvages de *P. aeruginosa.* Une gamme a été effectuée afin de définir la concentration minimale permettant une inhibition totale de ces souches et la valeur limite est de 1,5 mg/L.

L'ajout d'antibiotiques n'avait aucune influence sur l'expression des activités enzymatiques bactériennes sur le milieu chromogénique. Les groupes de germes étaient aussi bien séparés et identifiés sur milieu CPS ID 3 témoin que sur les milieux comprenant les antibiotiques tels que décrit ci dessus.

### b) Screening des inhibiteurs de β-lactamases

Trois inhibiteurs de β-lactamases (IBL), à savoir l'acide clavulanique, le tazobactam, et le sulbactam ont été utilisés. Chacun a été l'objet d'une gamme, en présence de cefotaxime, afin de déterminer la concentration optimale pour inhiber les souches BLSE positives, sans gêner la croissance des souches Case HN.

L'acide clavulanique apparaissait comme l'IBL le plus efficace en présence de cefotaxime. Le tazobactam et le sulbactam nécessitaient des concentrations supérieures à 2 mg/L pour inhiber les souches BLSE positives, alors que l'acide clavulanique était plus actif à des concentrations nettement inférieures et ce sur une plage d'utilisation large (de 0,1 à 8 mg/L lorsqu'il est utilisé en association avec le cefotaxime).

Chaque antibiotique (cefotaximine, ceftazidimine, ceftriaxone, cefpodoxime, aztreonam) a été testé en présence d'une gamme d'acide clavulanique afin de définir les combinaisons les plus efficaces. Les combinaisons retenues sont répertoriées dans le tableau IV ci-dessous.

**Tableau IV. Associations retenues d'antibiotiques et d'acide clavulanique (AC), inhibant les souches d'entérobactéries testées BLSE-positives, mais laissant pousser les souches Case HN positives**

| Cefotaxime | 2 mg/L | + AC | 0,1 mg/L |
|---|---|---|---|
| Ceftazidime | 2,5 mg/L | + AC | 2 mg/L |
| Ceftriaxone | 2 mg/L | + AC | 0,25 mg/L |
| Cefpodoxime | 9 mg/L | + AC | 0,25 mg/L |
| Aztreonam | 1 mg/L | + AC | 0,5 mg/L |

Les résultats obtenus en utilisant une bi-boîte contenant d'un côté l'antibiotique seul et de l'autre côté l'association antibiotique + acide clavulanique sont répertoriés dans le tableau V.

**Tableau V**

| | Antibiotique seul | Antibiotique + acide clavulanique |
|---|---|---|
| *E. coli* sauvage | | |
| *E. coli* BLSE positif | Colonies roses | |
| *E. coli* Case HN positif | Colonies roses | Colonies roses |
| *Proteeae* sauvage | | |
| *Proteeae* BLSE positif | Colonies marron | |
| *Proteeae* Case HN positif | Colonies marron | Colonies marron |
| KESC sauvage | | |
| KESC BLSE positif | Colonies vertes | |
| KESC Case HN positif | Colonies vertes | Colonies vertes |

L'ajout d'inhibiteurs de β-lactamases n'avait aucune influence sur l'expression des activités enzymatiques bactériennes sur le milieu chromogénique. Les groupes de germes étaient aussi bien séparés et identifiés sur milieu CPS ID 3 témoin que sur les milieux comprenant des inhibiteurs de β-lactamases.

### c) Combinaison d'antibiotiques

Compte tenu des activités relatives des antibiotiques et de l'acide clavulanique, il a été associé
- le cefotaxime (antibiotique actif sur les souches BLSE positives en combinaison avec la plus faible concentration d'acide clavulanique),
- la ceftazidime (antibiotique actif sur les souches sauvages de *P. aeruginosa*), et
- l'acide clavulanique,
de façon à pouvoir inhiber d'une part les souches sauvages (y compris celles du bacille pyocyanique), et les souches BLSE positives des entérobactéries testées par l'ajout de l'IBL.

Une telle association a été testée sur les souches de *P. aeruginosa,* cette espèce est naturellement résistante au cefotaxime mais sensible à la ceftazidime. En associant 1,5 mg/L de CAZ (ceftazidime), 1 mg/L de CTX (cefotaxime) et 0,25 mg/L d'AC (acide clavulanique), toutes les souches sauvages et les souches BLSE positives des entérobactéries testées ont été inhibées et ne poussait que l'ensemble des souches Case HN et les souches BLSE positives de *P aeruginosa.* Il s'agit d'une bi-boîte avec d'un côté la CAZ seule et de l'autre le CTX plus l'acide clavulanique.

L'ajout de ces combinaisons d'antibiotiques n'avait aucune influence sur l'expression des activités enzymatiques bactériennes sur le milieu chromogénique. Les groupes de germes étaient aussi bien séparés et identifiés sur milieu CPS ID 3 témoin que sur les milieux comprenant de telles combinaisons d'antibiotiques.

### d). Essai de colorant

Le milieu selon l'invention a également été mis en oeuvre pour une utilisation en bi-boîte, l'un des côtés contenant un antibiotique, et le second un autre antibiotique ou une combinaison d'antibiotiques. Etant donné que la même base de milieu CPS ID 3 est utilisée de part et d'autre, ces 2 côtés étaient différenciés par la présence d'un colorant.

Le colorant testé, le bleu d'Evans, donne au milieu une couleur verte. La coloration permettait de différencier aisément les 2 côtés de la bi-boîte, sans affecter la fertilité du milieu, ni gêner la lecture des activités enzymatiques des colonies. Après avoir effectué une gamme de bleu d'Evans, ajouté avant ou après autoclavage, les valeurs retenues étaient les suivantes :
- 1,5 ou 2 mg/L si le colorant est ajouté après autoclavage
- entre 2 et 5 mg/L s'il est ajouté avant autoclavage.

### e). Inhibition des bactéries Gram-positif

Les BLSE sont un mécanisme de résistance aux β-lactamines, retrouvé uniquement chez les bacilles Gram-négatif, il convient donc d'inhiber les bactéries Gram-positif par le milieu. Deux antibiotiques, le linezolide et la vancomycine ont été utilisés dans le milieu selon l'invention en vue d'inhiber les bactéries Gram-positif sensibles.

Pour la vancomycine, dans les conditions testées, une concentration comprise entre 2 et 32mg/l et notamment 2 à 5mg/l permet l'inhibition des bactéries à Gram positif sensibles sans interférer avec la détection des bactéries BLSE.

Pour le linezolide, dans les conditions testées, une concentration comprise entre 2 et 64mg/l et notamment 4 à 16mg/l permet l'inhibition des bactéries à Gram positif sensibles sans interférer avec la détection des bactéries BLSE.

L'inhibition des bactéries Gram positif permettait d'améliorer la détection des bactéries BLSE dans des prélèvements polymicrobiens et la spécificité de leur coloration.

### f) Inhibition des levures

Le milieu selon l'invention peut également comprendre des antifongiques afin d'inhiber la présence éventuelle de levures qui pourraient pousser sur le milieu et qui pourraient gêner la croissance des germes.

Deux antifongiques ont donc été testés : le voriconazole et l'amphotéricine B.

Pour l'amphotéricine B, dans les conditions testées, une concentration comprise entre 1 et 32mg/l et notamment 2 à 8mg/l permet l'inhibition des levures sensibles sans interférer avec la détection des bactéries BLSE.

Pour le voriconazole, dans les conditions testées, une concentration comprise entre 1 et 64mg/l et notamment 4 à 16mg/l permet l'inhibition des bactéries à Gram positif sensibles sans interférer avec la détection des bactéries BLSE.

L'inhibition des levures permettait d'améliorer la détection des bactéries BLSE dans des prélèvements polymicrobiens et la spécificité de leur coloration.

### 6. Conclusion

Ces résultats démontrent que le milieu selon l'invention permet l'isolement et l'identification présomptive des bactéries productrices de BLSE, en les différenciant des souches productrices de céphalosporinase à haut niveau. L'utilisation d'une bi-boîte contenant d'un côté une céphalosporine seule, et de l'autre une association céphalosporine/acide clavulanique, en base CPS ID 3, est particulièrement intéressante.

### EXEMPLE 2

Ce deuxième exemple est basé sur la détection phénotypique des BLSE utilisant la réduction de susceptibilité aux antibiotiques et la sensibilité des Case HN aux associations avec la tobramycine ou la cloxacilline ou la di-cloxacilline et présente l'utilisation d'une céphalosporine mentionnée ci-dessus (CTX, CAZ, CPD, CRO, ATM) en combinaison avec un composé inhibant les céphalosporinases (cloxacilline, di-cloxacilline et la tobramycine). Un tel milieu permet l'inhibition des bactéries ayant une céphalosporinase « naturelle », la plus part de celles ayant uniquement une céphalosporinase à haut niveau (Case HN), tout en permettant la croissance des bactéries BLSE.

### 1. Choix des souches

Dans le cadre des manipulations effectuées, pour l'évaluation de l'activité des antibiotiques actifs sur les bacilles gram-négatif, différentes espèces d'entérobactéries (*Escherichia coli, Enterobacter aerogenes, Klebsiella pneumoniae, Proteus mirabilis*) et de bacilles gram-négatif non fermentants (*Pseudomonas aeruginosa*) susceptibles de produire des BLSE ont été employées. Dans les essais sont comparées des souches BLSE positives, des souches productrices de céphalosporinase haut niveau (Case HN) et des souches sauvages.

### 2. Préparation du milieu

Le milieu utilisé était un milieu CPS ID3 (43541) comprenant en outre
- la ceftazidime à 2.5 mg/L et la tobramycine à 2 mg/L (milieu A) ou
- le ceftriaxone à 2 mg/L et la cloxacilline à 150 mg/L (milieu B) ou
- cefpodoxime à 2 mg/L et di-cloxacilline à une concentration comprise entre 500 et 1000 mg/L (milieu C)

L'eau osmosée est ajoutée et le tout est homogénéisé et mis à fondre au bain-marie à 100°C. Le milieu de base est réparti en flacons, en nombre correspondant au nombre total de milieux à tester lors de la manipulation. Les flacons sont ensuite autoclavés 15 mn à 121°C. Les milieux sont ramenés et maintenus en surfusion à 55 ± 3°C au bain-marie, afin d'ajouter stérilement les additifs thermolabiles (stérilisés préalablement par filtration sur 0,22 µm).

Les milieux sont ensuite coulés en boites de 35 mm de diamètre et laissés sur une surface plane pour qu'ils puissent reprendre en masse. Puis la surface des géloses est séchée sous hotte à flux laminaire pendant 30 mn.

### 3. Ensemencement des milieux

Cette étape est réalisée telle que décrite dans l'exemple 1

### 4. Lecture des milieux

Cette étape est réalisée telle que décrite dans l'exemple 1

### 5. Résultats :

Le milieu A comprenant la ceftazidime et la tobramycine permettait l'inhibition de toutes les souches sauvage et toutes les Case HN testées. Seules les souches BLSE positives étaient détectées sur ce milieu.

Le milieu B comprenant le ceftriaxone et la cloxacilline permettait d'inhiber toutes les Case HN et toutes les souches sauvages sauf *P aeruginosa* Case HN et sauvage, et ne poussait que la majorité des souches BLSE positives.

Le milieu C comprenant la cefpodoxime et la di-cloxacilline permettait l'inhibition de toutes les souches sauvages et la majorité des Case HN sans affecter la croissance des souches BLSE positives.

## Revendications

1. Utilisation d'un milieu de culture pour distinguer:
• un premier groupe de bactéries *E. coli* BLSE ;
• un deuxième groupe de bactéries KESC BLSE ;
• un troisième groupe de bactéries non résistantes aux beta-lactamines ;
ledit milieu de culture comprenant :
• un premier substrat permettant la détection d'une activité enzymatique beta-glucuronidase ou beta-galactosidase ;
• un deuxième substrat permettant la détection d'une activité beta-glucosidase ou tryptophanase ou désaminase ;
• la cefpodoxime ;
• la cloxacilline.

2. Utilisation, selon la revendication 1, d'un milieu de culture pour distinguer :
• un premier groupe de bactéries *E*. *coli* BLSE ;
• un deuxième groupe de bactéries KESC BLSE ;
• un troisième groupe de bactéries, non résistantes aux beta-lactamines et/ou aux céphalosporines ;
• un quatrième groupe de bactéries *Proteeae* BLSE ;
ledit milieu de culture comprenant :
• un premier substrat permettant la détection d'une activité enzymatique beta-glucuronidase ou beta-galactosidase ;
• un deuxième substrat permettant la détection d'une activité beta-glucosidase ;
• la cefpodoxime,
• la cloxacilline,
• la vancomycine,
• l'amphothéricine B ;
• un troisième substrat permettant la détection d'une activité désaminase.

3. Utilisation d'un milieu de culture, selon la revendication 2, ledit milieu de culture comprenant en outre de la cefsulodine.

## Patentansprüche

1. Verwendung eines Kulturmediums, um zwischen den Folgenden zu unterscheiden:
• einer ersten Gruppe von ESBL-*E*.*coli-*Bakterien;
• einer zweiten Gruppe von ESBL-KESC-Bakterien;
• einer dritten Gruppe von nicht gegen beta-Lactamine resistenten Bakterien;
wobei das Kulturmedium Folgendes umfasst:
• ein erstes Substrat, das den Nachweis einer beta-Glucuronidase- oder beta-Galactosidase-Enzymaktivität gestattet;
• ein zweites Substrat, das den Nachweis einer beta-Glucosidase- oder Tryptophanase- oder Desaminaseaktivität gestattet;
• Cefpodoxim;
• Cloxacillin.

2. Verwendung nach Anspruch 1 eines Kulturmediums, um zwischen den Folgenden zu unterscheiden:
• einer ersten Gruppe von ESBL-*E*.*coli-*Bakterien;
• einer zweiten Gruppe von ESBL-KESC-Bakterien;
• einer dritten Gruppe von nicht gegen beta-Lactamine und/oder Cephalosporine resistenten Bakterien;
• einer vierten Gruppe von ESBL-*Proteeae-*Bakterien;
wobei das Kulturmedium Folgendes umfasst:
• ein erstes Substrat, das den Nachweis einer beta-Glucuronidase- oder beta-Galactosidase-Enzymaktivität gestattet;
• ein zweites Substrat, das den Nachweis einer beta-Glucosidaseaktivität gestattet;
• Cefpodoxim,
• Cloxacillin,
• Vancomycin,
• Amphothericin B;
• ein drittes Substrat, das den Nachweis einer Desaminaseaktivität gestattet.

3. Verwendung eines Kulturmediums nach Anspruch 2, wobei das Kulturmedium weiterhin Cefsulodin umfasst.

## Claims

1. Use of a culture medium for distinguishing:
- a first group of ESBL *E.coli* bacteria
- a second group of ESBL KESC bacteria
- a third group of bacteria that is not resistant to beta-lactamines
the culture medium comprising:
- a first substrate for detecting a beta-glucuronidase or beta-galactosidase enzymatic activity
- a second substrate for detecting a beta-glucosidase or tryptophanase or desaminase activity
- cefpodoxime
- cloxacillin.

2. Use, according to claim 1, of a culture medium for distinguishing:
- a first group of ESBL *E*.*coli* bacteria
- a second group of ESBL KESC bacteria
- a third group of bacteria that is not resistant to beta-lactamines and/or to cephalosporins
- a fourth group of ESBL *Proteeae* bacteria
the culture medium comprising:
- a first substrate for detecting a beta-glucuronidase or beta-galactosidase enzymatic activity
- a second substrate for detecting a beta-glucosidase activity
- cefpodoxime
- cloxacillin
- vancomycin
- amphotericin B
- a third substrate for detecting a desaminase activity

3. Use of a culture medium, according to claim 2, the culture medium comprising furthermore cefsulodine
